# EUROPEAN PATENT APPLICATION

(11) **EP 2 161 256 A1**
(43) Date of publication of application: **10.03.2010**
(21) Application number: 08163840.5
(22) Date of filing: 08.09.2008
(51) Int. Cl.: C07D 207/46

(54) **Compound containing carboxylate ester and N2S2 ligand bi-functional groups and manufacturing method thereof**

(71) Applicant: Atomic Energy Council - Institute of Nuclear Energy Research, Taoyuan County (TW)
(72) Inventor: Liu, Show-Wen, 511, Changhua County (TW); Lin, Tsyh-Lang, 334, Taoyuan County (TW); Lin, Cheng-Hsien, 100, Taipei City (TW); Luo, Tsai-Yueh, 325, Taoyuan County (TW); Shen, Lie-Hang, 320, Taoyuan County (TW); Chen, Haw-Jan, 330, Taoyuan County (TW); Hsu, Cheng-Fang, 351, Miaoli County (TW)
(74) Representative: Lermer, Christoph

(57) **Abstract**

A compound containing carboxylate ester and N2S2 ligand bi-functional groups and a manufacturing method thereof are disclosed. The S in the N2S2 ligand of the compound containing carboxylate ester and N2S2 ligand bi-functional groups includes a protective group so as to avoid to be oxidized and easy storage- In a complex reaction, the protective group is automatically released As to the active carboxylate ester, it is for reacting with compounds having amino groups such as amines, amino acids, peptides, or protein etc while the N2S2 ligand is for bonding with technetium or rhenium so as to form neutral complex. The compound containing carboxylate ester and N2S2 ligand bi-functional groups is applied to radiopharmaceuticals such as contrast agents for tissues and target agents.

## Description

### BACKGROUND OF THE INVENTION

The present invention relates to a compound containing two functional groups and a manufacturing method thereof, especially to a compound containing carboxylate ester and N2S2 ligand bi-functional groups and a manufacturing method thereof that react with target material with amino-group as well as technetium or rhenium so as to form radiopharmaceuticals to be applied to contrast agents for tissues, target agents for diseases, or diagnosis devices.

The human cells have specific receptors on their surface so as to bind corresponding amines, amino acids, peptides, or protein. By means of such specificity, the compounds with these amino groups are labeled with radioactive nuclides. Once the compounds containing these amino groups entering human bodies, they are brought to specific organs or tissues so as to achieve purposes such as diagnostic imaging or disease treatment. For example, apoptosis is highly related to treatment of a plurality of diseases.

Thus Annexin-V labeled with radionuclides applied to research of apoptosis has received a lot of attention. If the protein or peptide is going to be labeled with technetium-99m (Tc-99m), the compound containing two functional groups is used for labeling protein with Tc-99m. S-Hynic is a common compound having two functional groups to be used. Besides an active carboxylic ester group that reacts with protein or peptides to generate a strong amide linkage, it includes pyridinyl and hydrazo groups that react with Tc-99m. However, S-Hynic is photosensitive substance so that it is light degraded. Moreover, the S-Hynic has low valency so that it requires addition of auxiliary substance such as tricine and this is not convenient in use. Thus there is a need to find other compounds containing two functional groups with stable physical properties and convenient to use.

In order to overcome above shortages of conventional compounds containing two functional groups, the present invention provides a compound containing carboxylate ester and N2S2 ligand bi-functional groups and a manufacturing method thereof. The compound containing carboxylate ester and N2S2 ligand bi-functional groups not only reacts with alcohols and amines but also with ReO³⁺ or TcO³⁺ to be applied to protein and peptide labeling with technetium or rhenium. Furthermore, the compound containing carboxylate ester and N2S2 ligand bi-functional groups has feature of photostability so that it is easy to be operated and used. Moreover, there is no need to add auxiliary cheating agents during the processes so that both reaction processes and the cost are reduced.

### SUMMARY OF THE INVENTION

Therefore it is a primary object of the present invention to provide a compound containing carboxylate ester and N2S2 ligand bi-functional groups and a manufacturing method thereof. The compound containing carboxylate ester and N2S2 ligand bi-functional groups not only reacts with alcohols and amines but also bonds with ReO³⁺ or TcO³⁺ so as to be applied to labeling of proteins and peptides with Tc or Re.

It is another object of the present invention to provide a compound containing carboxylate ester and N2S2 ligand bi-functional groups and a manufacturing method thereof. The compound containing carboxylate ester and N2S2 ligand bi-functional groups has photostability so that it is easy to be operated and used under environment with light.

It is a further object of the present invention to provide a compound containing carboxylate ester and N2S2 ligand bi-functional groups and a manufacturing method thereof. While using the compound containing carboxylate ester and N2S2 ligand bi-functional groups, there is no need to add auxiliary chelating agents during the processes so that both reaction processes and the cost are reduced.

In order to achieve above objects, the compound containing carboxylate ester and N2S2 ligand bi-functional groups of the present invention includes: 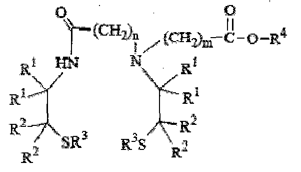 wherein R¹ =H, CH₃; R² =H, CH₃; R³ =CPh₃, CH₂C₆H₄OCH₃, COC₆H₅; 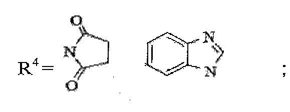 n = 1∼3; and m = 1∼9

The manufacturing method of the compound containing carboxylate ester and N2S2 ligand bi-functional groups comprising the steps of:
(1) reacting NH₂CR¹₂CR²₂SH with a protective agent to form NH_{z}CR¹₂CR²₂SR³
(2) carrying out an amidation reaction between NH₂CR¹₂CR²₂SR³ and X(CH₂)ₙCOX 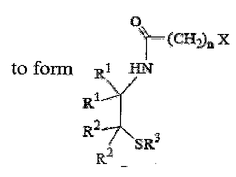
(3) undergoing a substitution reaction between 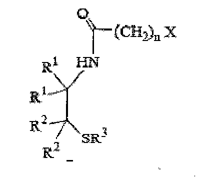 and NH₂CR¹₂CR²₂SR³ to form 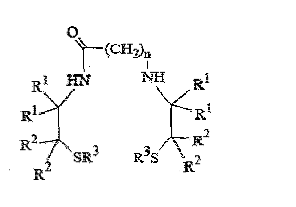
(4) carrying out a substitution reaction between 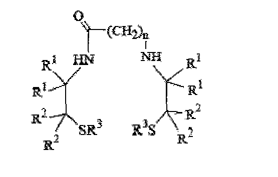 and Br(CH₂)mCOOH to form 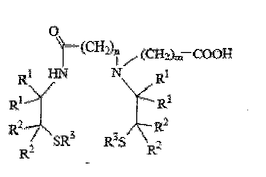
(5)dehydrating N,N'-dicyclohexyldicarbodiimide to get 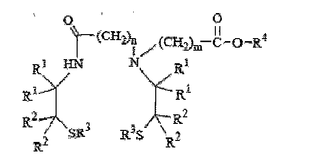
wherein R¹ =H, CH₃; R² =H, CH₃; R³ =CPh₃, CH₂C₆H₄OCH₃, COC₆H₅; 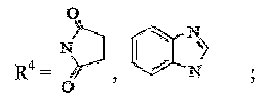 n =1∼3; m =1∼9 while X is halogen atom

### BRIEF DESCRIPTION OF THE DRAWINGS

The structure and the technical means adopted by the present invention to achieve the above and other objects can be best understood by referring to the following detailed description of the preferred embodiments and the accompanying drawings, wherein
Fig. 1 is a schematic drawing showing manufacturing processes of an embodiment of a compound containing carboxylate ester and N2S2 ligand bi-functional groups according to the present invention;
Fig. 2 is a schematic drawing showing manufacturing processes of SOCTA, and SOCTA labeled with amines and radioactive nuclides according to the present invention;

### DETAILED DESCRIPTION OF THE PREFFERED EMBODIMENT

A compound containing carboxylate ester and N2S2 ligand bi-functional groups according to the present invention includes: 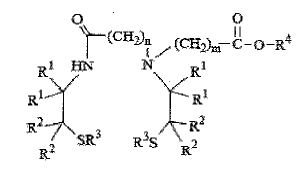 wherein R¹=H, CH₃ ; R²=H, CH₃ ; R³=CPh₃, CH₂C₆H₄OCH₃, COC₆H₅ ; 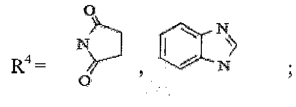 n = 1∼3; and m = 1∼9. while when n and m=1, and 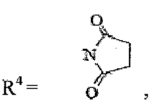 the compound containing carboxylate ester and N2S2 ligand bi-functional groups is Succinimidyl 3,6-diaza-5-oxo-3-[2-((triphenylmethyl)thio)ethyl] -8-[(triphenylmethyl)thio] octanoate, abbreviated as SOCTA.

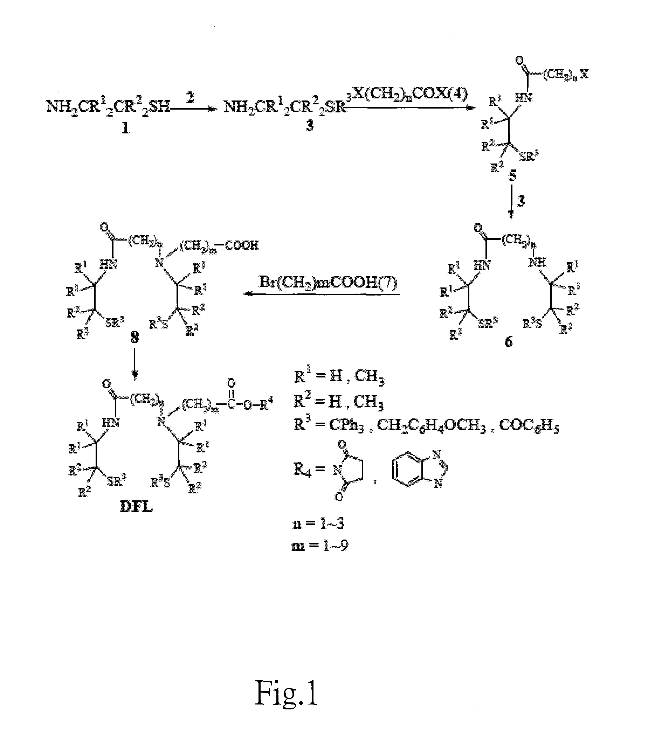

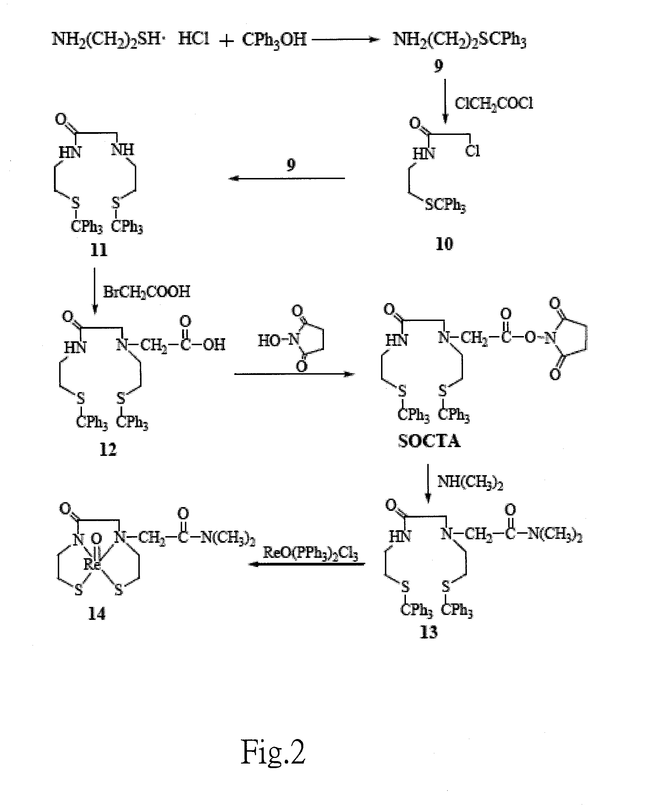

A manufacturing method of the compound containing carboxylate ester and N2S2 ligand bi-functional groups according to the present invention includes the following steps (as shown in Fig. 1):
(1) reacting NH₂CR¹₂CR²₂SH with a protective agent to form NH₂CR¹₂CR²₂SR³ ;
(2) carrying out an amidation reaction between NH₂CR¹₂CR²₂SR³ and X(CH₂)ₙCOX to form 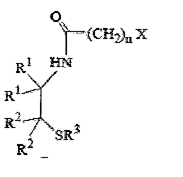
(3) undergoing a substitution reaction between 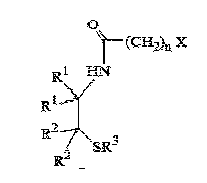 and NH₂CR¹₂CR²₂SR³ to form 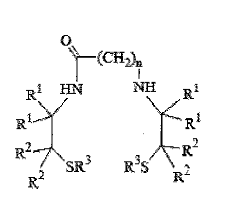
(4) carrying out a substitution reaction between 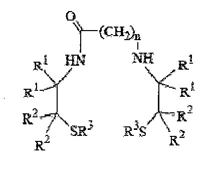 Br(CH₂)ₘCOOH and to form 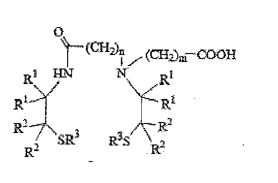
(5) dehydrating N,N'-dicyclohexyldicarbodiimide to get 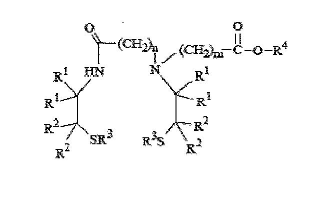
wherein R¹ =H, CH₃; R² =H, CH₃; R³ =CPh₃, CH₂C₆H₄OCH₃, COC₆H₅; 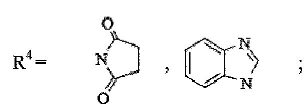 n =1∼3; m =1∼9 while X is halogen atom
In the step (1), the protective agent is triphenyl methanol. The step (1) further includes a step to dissolve in trichloromethane and then apply a heat reflow step in which boron trifluoride ethyl etherate complex is added as a catalyst. The step (2) further consists of a step of washing the organic phase and condensation under reduced pressure after the step of dissolving in trichloromethane. The step (3) further includes a step of dissolving in dichloromethane, adding triethylamine followed by a heat flow process and liquid chromatography for purification while the step (4) further includes a heat reflow step after addition of bromoacetic acid, triethylamine and acetonitrile, condensation under reduced pressure, and liquid chromatography for purification. The step (5) includes a step of adding N-hydroxysuccinimide, 1, 3-dicyclohexylcarbodiimide and tetrahydrofuran to form N,N'-dicyclohexyldicarbodiimide.
In the step(5), when n and m = 1 and 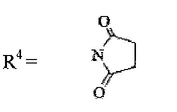 , the ompound containing carboxylate ester and N2S2 ligand bi-functional groups is Succinimidyl 3,6-diaza-5-oxo-3-[2-((triphenylmethyl)thio)ethyl] -8-[(triphenylmethyl)thio] octanoate, abbreviated as SOCTA.
Take a compound with bi-funclional groups-SOCTA (uccinimidyl 3,6-diaza-5-oxo-3-[2-((triphenylmethyl)thio)ethyl]-8-[(triphenylmethyl)thio] octanoate) as an example, refer to Fig. 2, a synthesis method of SOCTA, the amidation reaction of SOCTA with amines and the following complex reaction are described:

### (1) synthesis of 1, 2-[(Triphenylmethyl)thio]ethylamine 9

Take 10 g (88.4 mmol) 2-thioethylamine hydrochloride, 22 g (85 mmol) triphenylmethanol, and 14 mL (99.7 mmol) triethylamine, all dissolve in 100 mL trichloromethane. Then apply a heat reflow step at 75°C and slowly drop 30 mL (239 mmol) boron trifluoride ethyl ether complex into the solution and continue the heat reflow step for four hours. Next perform the condensation under reduced pressure, add methanol to dissolve, and condensed again. Add sodium bicarbonate solution and stir the solution. Then a white solid precipitate is obtained. After vacuum filtering, wash the obtained solid and dried to get 27.9 g (99%) solid compound 9.
IR (neat) ν 3381 (NH₂) cm⁻¹. ¹H NMR (CDCl₃) δ 7.42 (m, 3 H, Ph), 7.30 (m, 12 H, Ph), 2.58 (t, J = 6.6 Hz, 2 H, CH₂N), 2.32 (t, J = 6.6 Hz, 2 H, CH₂S), 1.45 (br, 2 H, NH₂). ¹³C NMR (CDCl₃) δ 144.80, 192.52, 127.81 and 126.60 (Ph), 66.51 (CPh), 40.94 (CH₂N), 36.09 (CH₂S). MS m/z 319 (M⁺), 243 (M⁺ - C₆H₅ + 1).

### (2) synthesis of N-[2-((Triphenylmethyl)thio) ethyl]chloroacetamide 10

Take 10.63 g, (33.3 mmol) compound 9 and 5.6 mL (39.9 mmol) triethylamine, dissolve them in 80mL trichloromethane. Slowly drop solution of 3.18 mL (39.9 mmol) chloroacetyl chloride dissolved in 10 mL trichloromethane into the mixture, cooling in ice. After dripping off, stir the solution at room temperature for two hours. Wash the organic phase by following solutions in sequence: 1N Hydrochloric acid solution HCl (120 mL), saturated sodium carbonate aqueous solution (100 mL) and water (100 mL). After dehydration of the organic phase with Na₂SO₄, condense under reduced pressure to get yellow oil product 10 (12.67g, 96%).
IR (neat) ν 3413 and 3306 (NH), 1662 (CO) cm⁻¹. ¹H NMR (CDCl₃) δ 7.41 (m, 3 H, Ph), 7.24 (m, 12 H, Ph), 6.48 (br, 1 H, NH), 3.97 (s, 2 H, CH₂Cl), 3.12 (q, J = 6.3 Hz, 2 H, CH₂N), 2.43 (t, J = 6.3 Hz, 2 H, CH₂S). ¹³C NMR (CDCl₃) δ 165.63 (CO), 144.47, 129.48, 127.97 and 126.81 (Ph), 66.52 (CPh), 42.54 (CH₂Cl), 38.35 (CH₂N), 31.67 (CH₂S). MS m/z 397 and 395 (M⁺), 243 ((CPh₃)⁺).

### (3) synthesis of N-[2-((Triphenylmethyl)thio)ethyl] [2-((triphenylmethyl)thio)ethyl-amino]acetamide 11

Dissolve 12.7 g (32 mmol) the compound 10 and 10.2 g (32 mmol) the compound 9 into 60 mL dichloromethane. Then add 6.7 mL (48 mmol) triethylamine followed by a heat flow process for two days. After cooling, wash with 60 mL Sodium hydrogen carbonate aqueous solution, and wash again with 60 mL water, take the organic layer. The organic phase is dried by Na₂SO₄, condensed and then purified by liquid chromatography (SiO₂, ethyl acetate : hexane = 1 : 1) to get light yellow oil product 11 (12.9 g, 59.9%).
IR (neat) ν 3330 (NH), 1670 (CO) cm⁻¹. ¹H NMR (CDCl₃) δ 7.42 (m, 4 H, HNCO and Ph), 7.20 (m, 12 H, Ph), 3.07 (m, 4 H, CH₂NCO and CH₂CO), 2.38 (m, 6 H, CH₂NHCH₂CO and CH₂S), 1.94 (br, 1 H, NHCH₂CO). ¹³C NMR (CDCl₃) δ 170.84 (CO), 144.61, 129.47, 127.88 and 126.69 (Ph), 66.72 and 66.65 (CPh₃), 51.62 (CH₂CO), 48.19 (CH₂NHCH₂CO), 37.70 (CH₂NHCO), 32.12 and 31.97 (CH₂S). MS m/z 243 ((CPH₃)⁺).

### (4)synthesis of 3,6-Diaza-5-oxo-3-[2-((triphenylmethyl)thio)ethyl]-8-[(triphnylmethyl) thio]octanoic acid 12

Take 10.1 g (14.9 mmol) compound 11, 2.2 g (15.8 mmol) bromoacetic acid, 3.13 mL (22.3 mmol) triethylamine and 30 mL acetonitrile and perform a heat reflow step at 85°C overnight. After cooling, condensation under reduced pressure, dissolve the residual in 80 mL dichloromethane and wash with 80 mL water, remove the water phase. The organic phase is dehydrated by Na₂SO₄, condensed and purified by liquid chromatography (SiO₂, ethylacetate : hexane = 1 : 1) to get light yellow oil product 12 (6.5 g, 56.1%).
IR (neat) ν 3327 (NH), 1726 and 1634 (CO) cm⁻¹. ¹H NMR (CD₃OD) δ 7.40 (m, 3 H, Ph), 7.25 (m, 12 H, Ph), 3.21 (s, 2 H, CH₂), 3.11 (s, 2 H, CH₂), 2.30 (t, J = 6.6 Hz, 2 H, CH₂NH), 2.52 (t, J = 6.6 Hz, 2 H, CH₂N), 2.34 (t, J = 6.6 Hz, 4, H, CH₂S). ¹³C NMR (CD₃OD) δ 173.89 and 172.97 (CO), 146.11, 130.72, 128.96, 127.87 and 127.81 (Ph), 68.09 and 67.84 (CPh₃), 59.0, 55.86 and 55.13 (CH₂), 39.12 (CH₂NH), 32.70 and 31.01 (CH₂S). MS m/z 243 ((CPh₃)⁺).

### (5)synthesis of Succinimidyl 3,6-diaza-5-oxo-3-[2-((triphenylmethyl)thio)ethyl] -8-[(triphenylmethyl)thio]octanoate (SOCTA)

Take 1.65 g (2.1 mmol) compound 12, add with 0.3 g (2.52 mmol) N-hydroxysuccinimide, 0.7 g (31.5 mmol)) 1, 3-dicyclohexylcarbodiimide, and 30 mL tetrahydrofuran, stir at room temperature overnight. Remove the solid percipitate by vacuum filtering and condense the solution under reduced pressure. The purify by liquid chromatography (SiO2, ethyl acetate) to get oil product SOCTA (1.79 g, 60.2%).
IR (neat) ν 3360 (NH), 1815, 1786, 1741 and 1674 (CO) cm⁻¹. ¹H NMR (CDCl₃) δ 7.37 (m, 4 H, NH and Ph), 7.21 (m, 12 H, Ph), 3.49 (s, 2 H, CH₂), 3.31 (s, 2 H, CH₂), 3.02 (q, J = 6.6 Hz, 2 H, CH₂NH), 2.75 (s, 4 H, CH₂CH₂CO), 2.58 (t, J = 6.6 Hz, 2 H, CH₂N), 2.36 (t, J = 6.6 Hz, 2 H, CH₂S), 2.31 (t, J = 6.6 Hz, 2 H, CH₂S). ¹³C NMR (CDCl₃) δ 169.68, 168.56 and 165.63 (CO), 144.68, 144.50 129.52, 129.47, 127.96, 127.88, 126.78 and 126.65 (Ph), 66.94 and 66.68 (CPh₃), 57.96, 53.31 and 52.41 (CH₂), 38.07 (CH₂NH), 31.89 and 29.93 (CH₂S), 25.50 (CH₂CH₂CO). MS m/z 243 ((CPh₃)⁺).
Refer to Fig. 2, the experiment shows SOCTA conjugating with amino groups and radioactive nuclides:

### (1)Synthesis of N,N-Dimethyl 3,6-diaza-5-oxo-3-[2-((triphenylmethyl)thio)ethyl] -8-[(triphenylmethyl)thio]octanamide 13

Dissolve 1.67 g (2.0 mmol) SOCTA and 10 mL (18.2 mmol) dimethyl amine in 30 mL trichloromethane and stir the solution at room temperature overnight. After being dried under reduced pressure, dissolve the residual with ethyl ether and purify by liquid chromatography (SiO₂, chloroform) to get oil product 13 (1.22 g, 80%).
IR (neat) ν 3341 (NH), 1673 and 1651 (CO) cm⁻¹. ¹H NMR (CDCl₃) δ 7.87 (t, J = 6.6 Hz, 1, NH), 7.21-7.06 (m, 30 H, Ph), 3.19 (s, 2 H, CH₂CO), 3.09 (s, 2 H, CH₂CO), 3.04 (q, J = 6.6 Hz, 2 H, CH₂NHCO), 2.82 (s, 3 H, NCH₃), 2.77 (s, 3, H, NCH₃), 2.64 (t, J = 6.6 Hz, 2 H, CH₂ CH₂NCH₂CO), 2.39 (t, J = 6.6 Hz, 2 H, CH₂CH₂NHCO), 2.26 (t, J = 6.6 Hz, 2 H, CH₂CHNCH₂CO). ¹³C NMR (CDCl₃) δ 171.17 and 169.67 (CO), 144.74, 144.70, 129.52, 129.49, 127.83, 126.63 and 126.60 (Ph), 66.69 and 66.54 (CPh), 58.39, 54.58, 53.97 and 37.98 (CH₂), 36.16 and 35.38 (NCH₃), 31.88 and 30.23 (CH₂). MS m/z 243 ((CPh₃)⁺).

### (2) synthesis of [N-(2-thioethyl)-N-(2-thioethyl)-N-(N,N-dimethy carbamoylmethyl) aminoacetimido] oxorhenium(V) 14

Dissolve 0.63 g (0.83 mmol) compound 13, 0.87 g (1.0 mmol) ReO(PPh₃)₂Cl₃, and 0.4 mL (2.9 mmol) triethylamine in 50 mL methanol and heat the solution at 55°C for 16 hours. After filtering, dry the solution under reduced pressure and dissolve the residue with trichloromethane. Wash with water and perform the liquid chromatography (SiO₂ , chloroform : methanol = 100 : 5) for purification to get reddish brown sticky product 14 (0.18 g, 45%).
IR (neat) ν 1639 and 1614 (CO), 961 (ReO) cm⁻¹. ¹H NMR (CDCl₃) δ 5.62 (d, J = 15.0 Hz, 1 H, NCOCH₂N), 4.83 (dd, J = 12.0 and 4.8 Hz, 1 H, CONCH₂CH₂), 4.66 (dd, J = 15.0 and 2.4 Hz, 1 H, NCOCH₂N), 4.32 (m, 1 H, COCH₂NCH₂CH₂), 3.76 (m, 1 H, COCH₂NCH₂CH₂), 3.60 - 3.30 (m, 3 H, CONCH₂CH₂), 3.26 - 3.05 (m, 3 H, COCH₂NCH₂CH₂), 2.89 (s, 3 H, CH₃), 2.72 (s, 3 H, CH₃), 2.42 (d, J = 17.1 Hz, 1 H, NCOCH₂N). ¹³C NMR (CDCl₃) δ 184.88 and 163.64 (CO), 68.29, 63.58, 54.28, 53.40, 47.98 and 40.69 (CH₂), 36.65 and 35.67 (CH₃). MS m/z 479 and 477 (M⁺).

The experiment showing conjugation of SOCTA with amino groups and radioactive nuclides proves that the SOCTA compound in the compound containing carboxylate ester and N2S2 ligand bi-functional groups according to the present invention not only reacts with amino acids, peptides or proteins but also bonds with radionuclides such as ReO³⁺ or TcO³⁺. Thus it can be applied to labeling of proteins/peptides with Tc/Re or disease treatment. Moreover, the compound containing carboxylate ester and N2S2 ligand bi-functional groups has photostability so that it is easy to be operated and used under environment with light. Furthermore, there is no need to add auxiliary chelating agents during the processes so that both reaction processes and the cost are reduced.

Additional advantages and modifications will readily occur to those skilled in the art. Therefore, the invention in its broader aspects is not limited to the specific details, and representative devices shown and described herein. Accordingly, various modifications may be made without departing from the spirit or scope of the general inventive concept as defined by the appended claims and their equivalents.

## Claims

1. A compound containing carboxylate ester and N2S2 ligand bi-functional groups comprising: 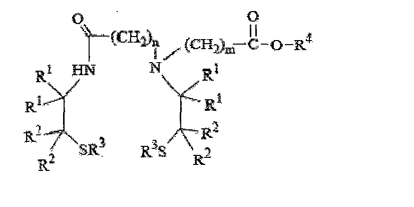 wherein R¹ =H, CH₃; R² =H, CH₃; R³ =CPh₃, CH₂C₆H₄OCH₃, COC₆H₅; 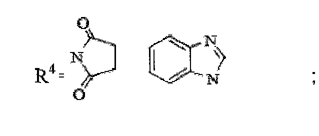 n =1∼3 and m =1∼9.

2. The compound as claimed in claim 1, wherein when both n and m=1, and R⁴= 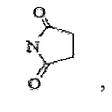 the compound is Succinimidyl 3,6-diaza-5-oxo-3-[2-((triphenylmethyl)thio)ethyl]-8-[(triphenylmethyl)thio]octanoate, abbreviated as SOCTA.

3. A manufacturing method of the compound containing carboxylate ester and N2S2 ligand bi-functional groups comprising the steps of:
(1) reacting NH₂R¹₂CR²₂SH with a protective agent to form NH₂R¹₂CR²₂SR³
(2) carrying out an amidation reaction between NH₂CR¹₂CR²₂SR³ and X(CH²)ₙCOX to form 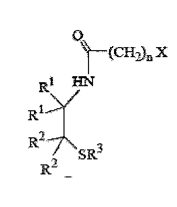
(3) undergoing a substitution reaction between 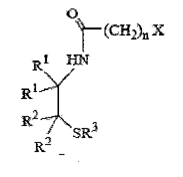 NH₂CR¹₂CR²₂SR³ and to form 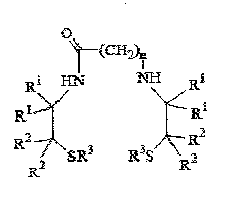
(4) carrying out a substitution reaction between 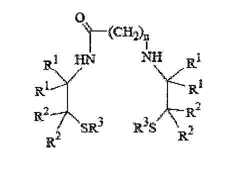 Br(CH₂)mCOOH and to form 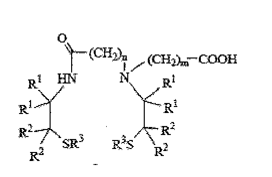
(5) dehydrating N,N'-dicyclohexyldicarbodiimide to get 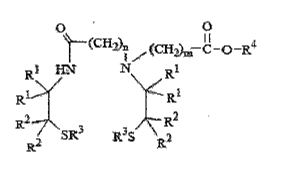
wherein R¹ =H, CH₃; R² =H, CH₃; R³ =CPh₃, CH₂C₆H₄OCH₃, COC₆H₅; 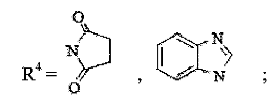 n =1∼3; m =1∼9 while X is halogen atom

4. The method as claimed in claim 3, wherein the protective agent is triphenyl methanol.

5. The method as claimed in claim 3, wherein the step (1) further comprising a heat reflow step after dissolving in trichloromethane.

6. The method as claimed in claim 5, wherein in the heat reflow step, boron trifluoride ethyl etherate complex is added as a catalyst.

7. The method as claimed in claim 3, wherein the step (2) further comprising a step of washing an organic phase and then being condensed under reduced pressure after dissolving in trichloromethane.

8. The method as claimed in claim 3, wherein the step (3) further comprising a step of dissolving in dichloromethane, adding triethylamine followed by a heat flow process and liquid chromatography for purification.

9. The method as claimed in claim 3, wherein the step (4) further comprising a eat reflow step after addition of bromoacetic acid, triethylamine and acetonitrile, then being condensed under reduced pressure, and purified by liquid chromatography.

10. The method as claimed in claim 3, wherein the step (3) further comprising a step of adding N-hydroxysuccinimide, 1, 3-dicyclohexylcarbodiimide and tetrahydrofuran to form N,N'-dicyclohexyldicarbodiimide.

11. The method as claimed in claim 3, wherein in the step (5), when both n and m=1, 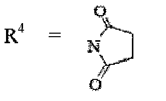 the compound containing carboxylate ester and N2S2 ligand bi-functional groups is Succinimidyl 3,6-diaza-5-oxo-3-[2-((triphenylmethyl)thio)ethyl]-8-[(triphenylmethyl)thio]octanoate, abbreviated as SOCTA.
